(19) Europäisches Patentamt
European Patent Office
Office européen des brevets

(11) Veröffentlichungsnummer: **0 019 153**
**B1**

(12) # EUROPÄISCHE PATENTSCHRIFT

(45) Veröffentlichungstag der Patentschrift: **10.11.82**

(51) Int. Cl.³: **C 07 D 249/08,** A 01 N 43/64

(21) Anmeldenummer: **80102370.6**

(22) Anmeldetag: **02.05.80**

(54) **Carbinolether, Verfahren zu ihrer Herstellung und diese enthaltende Fungizide.**

(30) Priorität: **10.05.79 DE 2918801**

(43) Veröffentlichungstag der Anmeldung: **26.11.80 Patentblatt 80/24**

(45) Bekanntmachung des Hinweises auf die Patenterteilung: **10.11.82 Patentblatt 82/45**

(84) Benannte Vertragsstaaten: **AT BE CH DE FR GB IT LI LU NL SE**

(56) Entgegenhaltungen: **DE-A-2 720 949**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Zeeh, Bernd, Dr., Thorwaldsenstrasse 5, D-6700 Ludwigshafen (DE)**
Erfinder: **Ammermann, Eberhard, Dr., Sachsenstrasse 3, D-6700 Ludwigshafen (DE)**
Erfinder: **Pommer, Ernst-Heinrich, Dr., Berliner Platz 7, D-6703 Limburgerhof (DE)**

**EP 0 019 153 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Carbinoläther, Verfahren zu ihrer Herstellung und diese enthaltende Fungizide

Die vorliegende Erfindung betrifft neue Carbinoläther, Verfahren zu ihrer Herstellung, Fungizide, die diese Wirkstoffe enthalten, sowie Verfahren zur Bekämpfung von Schadpilzen mit diesen Wirkstoffen.

Es ist bekannt, dass das 1-[2'-(2",4"-Dichlorphenyl)-2'-(2"-propenyloxy)]-äthyl-1H-imidazol (GB-C Nr. 1318590) fungizide Wirksamkeit hat.

Seine Wirkung, insbesondere gegen Mehltau- und Rostpilze, ist jedoch unzureichend. Daher ist es zur Bekämpfung von Schadpilzen im Pflanzenschutz wenig geeignet.

Es ist ferner bekannt, Azolylätherderivate, z.B. das 2-Allyloxy-1-(4-phenylphenoxy)-3,3-dimethyl-1-(1,2,4-triazol-1-yl)-butan als Fungizid zu verwenden (DE-A Nr. 2720949).

Es wurde nun gefunden, dass die neuen Carbinoläther der allgemeinen Formel (I)

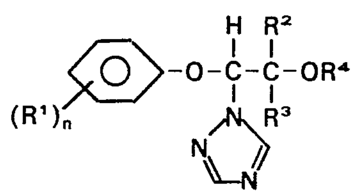

(I)

in welcher:

$R^1$ Halogen oder Phenyl

$R^2$ Alkyl mit 1 bis 6 C-Atomen oder gegebenenfalls durch Chlor substituiertes Phenyl

$R^3$ Alkyl mit 1 bis 6 C-Atomen, Vinyl, Äthinyl oder gegebenenfalls durch Chlor substituiertes Benzyl

$R^4$ Alkyl mit 1 bis 4 C-Atomen, den Allylrest, gegebenenfalls durch Chlor substituiertes Benzyl oder 3,3-Dimethylbutan-2-on-1-yl und

n 0, 1 oder 2 bedeutet sowie deren Salze und Metallkomplexverbindungen eine ausgezeichnete fungizide Wirksamkeit besitzen.

In der Formel (I) steht $R^1$ für Halogen beispielsweise Fluor, Chlor oder Brom oder für Phenyl.

$R^2$ steht beispielsweise für einen Alkylrest mit 1 bis 6 Kohlenstoffatomen, der unverzweigt sein kann z.B. Methyl, Äthyl, Propyl, Butyl oder Pentyl oder verzweigt sein kann beispielsweise Isopropyl oder tert.-Butyl. Ferner steht $R^2$ für einen unsubstituierten oder beispielsweise durch Chlor mono- oder disubstituierten Phenylrest.

$R^3$ steht z.B. für einen unverzweigten Alkylrest mit 1 bis 6 Kohlenstoffatomen beispielsweise Methyl, Äthyl, Propyl, Butyl oder Pentyl, z.B. für einen verzweigten Alkylrest mit 3 bis 6 Kohlenstoffatomen beispielsweise einen Isopropyl- oder Isobutylrest, für Vinyl, Äthinyl, oder z.B. für einen gegebenenfalls durch Chlor mono- oder disubstituierten Benzylrest.

$R^4$ steht für einen Alkylrest mit 1 bis 4 Kohlenstoffatomen der unverzweigt sein kann (z.B. einen Methyl-, Äthyl-, n-Propylrest) oder der verzweigt sein kann (beispielsweise einen Isopropyl-, einen 2-Methylpropyl-1-rest), für den Allylrest oder für gegebenenfalls durch Chlor substituiertes Benzyl oder 3,3-Dimethyl-butan-2-on-1-yl.

Die neuen Carbinoläther besitzen im Acetal-C-atom und — falls $R^2$ und $R^3$ unterschiedlich sind — im Carbinol-C-atom zwei Asymmetriezentren. Je nach der Beschaffenheit von $R^4$ kommen eventuell weitere Asymmetriezentren dazu. Mit üblichen Trennmethoden können einheitliche Diastereomere erhalten werden, die ebenfalls Gegenstand der Erfindung sind. Als Fungizide kann man sowohl die einheitlichen Diastereomeren wie auch die bei der Synthese üblicherweise anfallenden Gemische verwenden.

Die zur Synthese der neuen Carbinoläther als Ausgangsmaterialien notwendigen Carbinole der Formel (II),

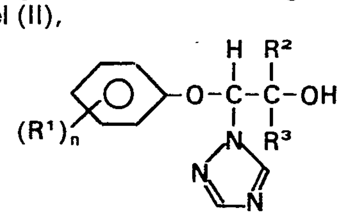

(II)

worin $R^1$, $R^2$, $R^3$ und n die oben erläuterten Bedeutungen haben, sind teilweise bekannt.

So offenbart die DE-A Nr. 2324010 die Herstellung des 1-(4'-Chlorphenoxy)-1-(1',2',4'-triazol-1'-yl)-2,3,3-trimethylbutan-2-ol durch eine Grignardreaktion nämlich die Umsetzung des Methylmagnesiumjodids mit 1-(4'-Chlorphenoxy)-1(1',2',4'-triazol-1'-yl)-3,3-dimethylbutan-2-on. Entsprechend können andere Ketone, wie sie z.B. aus den Deutschen Offenlegungsschriften Nrn 2201063 und 2705678 bekannt sind, mit Grignardreagentien der Formel $R^3$ Mg Hal, worin $R^3$ die oben genannten Bedeutungen hat und Hal für Chlor, Brom oder Jod steht, zu den Carbinolen der Formel (II) umgesetzt werden.

Carbinole der Formel (IV)

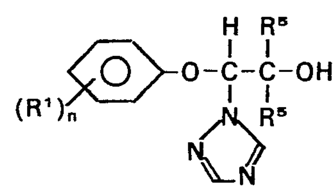

(IV)

worin $R^1$ und n die oben angegebenen Bedeutungen haben und $R^5$ für einen verzweigten oder unverzweigten Alkylrest mit 1 bis 6 Kohlenstoffatomen steht, können — wie gefunden wurde — dadurch hergestellt werden, dass man Ester der Formel (V)

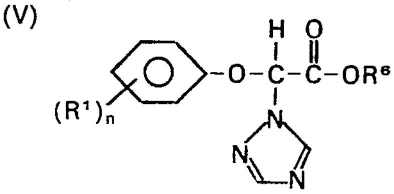

(V)

worin $R^1$ und n die oben genannten Bedeutungen haben und $R^6$ einen Alkylrest mit 1 bis 4 C-Atomen z.B. einen Methyl-, Äthyl-, Propyl- oder Butylrest bedeutet, mit Grignardreagentien der Formel (VI)

R$^5$ Mg Hal (VI)

worin R$^5$ die oben angegebene Bedeutung hat und Hal für Chlor, Brom oder Jod steht in Gegenwart inerter Lösungsmittel umsetzt.

Die Ester der Formel (V) sind bekannte Verbindungen. Ihre Synthese ist in der DE-A Nrn 2720654 und 2846127 beschrieben.

Für die Lösungsmittel, die Herstellung der Grignardreagentien, die Reaktionsbeschleuniger und die Reaktionstemperaturen sowie für die Isolierung der Carbinole gelten die aus der Literatur bekannten Bedingungen (vgl. Houben-Weyl, «Methoden der organischen Chemie», Thieme-Verlag Stuttgart 1973, Bd 13/2 a, S. 47 ff).

Als Ausgangssubstanzen für die Äthersynthese werden beispielsweise die folgenden Carbinole entsprechend der Formel (II) bzw. (IV) genannt:
1-(1',2',4'-Triazol-1'-yl)-1-(4'-chlorphenoxy)-2-methylpropan-2-ol,
1-(1',2',4'-Triazol-1'-yl)-1-(2',4'-dichlorphenoxy)-2-methylpropan-2-ol,
1-(1',2',4'-Triazol-1'-yl)-1-(4'-phenylphenoxy)-2-methylpropan-2-ol,
1-(1',2',4'-Triazol-1'-yl)-1-(4'-chlorphenoxy)-2-äthylbutan-2-ol,
1-(1',2',4'-Triazol-1'-yl)-1-(4'-phenylphenoxy)-2-äthylbutan-2-ol,
1-(1',2',4'-Triazol-1'-yl)-1-(4'-chlorphenoxy)-2-n-propylpentan-2-ol,
1-(1',2',4'-Triazol-1'-yl)-1-(phenoxy)-2-methylbutan-2-ol,
1-(1',2',4'-Triazol-1'-yl)-1-(4'-chlorphenoxy)-2-methylbutan-2-ol,
1-(1',2',4'-Triazol-1'-yl)-1-(4'-phenylphenoxy)-2-methylbutan-2-ol,
1-(1',2',4'-Triazol-1'-yl)-1-(4'-chlorphenoxy)-2-methylpentan-2-ol,
1-(1',2',4'-Triazol-1'-yl)-1-(4'-chlorphenoxy)-2,4-dimethylpentan-2-ol,
1-(1',2',4'-Triazol-1'-yl)-1-(phenoxy)-2,3,3-trimethylbutan-2-ol,
1-(1',2',4'-Triazol-1'-yl)-1-(4'-chlorphenoxy)-2,3,3-trimethylbutan-2-ol,
1-(1',2',4'-Triazol-1'-yl)-1-(2',4'-dichlorphenoxy)-2,3,3-trimethylbutan-2-ol,
1-(1',2',4'-Triazol-1'-yl)-1-(4'-phenylphenoxy)-2,3,3-trimethylbutan-2-ol,
1-(1',2',4'-Triazol-1'-yl)-1-(phenoxy)-2-äthyl-3,3-dimethylbutan-2-ol,
1-(1',2',4'-Triazol-1'-yl)-1-(4'-chlorphenoxy)-2-äthyl-3,3-dimethylbutan-2-ol,
1-(1',2',4'-Triazol-1'-yl)-1-(2',4'-dichlorphenoxy)-2-äthyl-3,3-dimethylbutan-2-ol,
1-(1',4'-Triazol-1'-yl)-1-(4'-phenylphenoxy)-2-äthyl-3,3-dimethylbutan-2-ol,
1-(1',2',4'-Triazol-1'-yl)-1-(phenoxy)-2-tert.-butylpentan-2-ol,
1-(1',2',4'-Triazol-1'-yl)-1-(4'-chlorphenoxy)-2-tert.-butylpentan-2-ol,
1-(1',2',4'-Triazol-1'-yl)-1-(2',4'-dichlorphenoxy)-2-tert.-butylpentan-2-ol,
1-(1',2',4'-Triazol-1'-yl)-1-(4'-phenylphenoxy)-2-tert.-butylpentan-2-ol,
1-(1',2',4'-Triazol-1'-yl)-1-(phenoxy)-2-tert.-butyl-4-methylpentan-2-ol,
1-(1',2',4'-Triazol-1'-yl)-1-(4'-chlorphenoxy)-2-tert.-butyl-4-methylpentan-2-ol,
1-(1',2',4'-Triazol-1'-yl)-1-(2',4'-dichlorphenoxy)-2-tert.-butyl-4-methylpentan-2-ol,
1-(1',2',4'-Triazol-1'-yl)-1-(4'-phenylphenoxy)-2-tert.-butyl-4-methylpentan-2-ol,
1-(1',2',4'-Triazol-1'-yl)-1-(phenoxy)-2-vinyl-3,3-dimethylbutan-2-ol,
1-(1',2',4'-Triazol-1'-yl)-1-(4'-chlorphenoxy)-2-vinyl-3,3-dimethylbutan-2-ol,
1-(1',2',4'-Triazol-1'-yl)-1-(2',4'-dichlorphenoxy)-2-vinyl-3,3-dimethylbutan-2-ol,
1-(1',2',4'-Triazol-1'-yl)-1-(4'-phenylphenoxy)-2-vinyl-3,3-dimethylbutan-2-ol,
1-(1',2',4'-Triazol-1'-yl)-1-(4'-chlorphenoxy)-2-äthinyl-3,3-dimethylbutan-2-ol,
1-(1',2',4'-Triazol-1'-yl)-1-(2',4'-dichlorphenoxy)-2-äthinyl-3,3-dimethylbutan-2-ol,
1-(1',2',4'-Triazol-1'-yl)-1-(phenoxy)-2-benzyl-3,3-dimethylbutan-2-ol,
1-(1',2',4'-Triazol-1'-yl)-1-(4'-chlorphenoxy)-2-benzyl-3,3-dimethylbutan-2-ol,
1-(1',2',4'-Triazol-1'-yl)-1-(2',4'-dichlorphenoxy)-2-benzyl-3,3-dimethylbutan-2-ol,
1-(1',2',4'-Triazol-1'-yl)-1-(4'-phenylphenoxy)-2-benzyl-3,3-dimethylbutan-2-ol,
1-(1',2',4'-Triazol-1'-yl)-1-(4'-chlorphenoxy)-2-phenylpropan-2-ol,
1-(1',2',4'-Triazol-1'-yl)-1-(4'-phenylphenoxy)-2-phenylpropan-2-ol,
1-(1',2',4'-Triazol-1'-yl)-1-(4'-phenylphenoxy)-2-phenylpropan-2-ol,
1-(1',2',4'-Triazol-1'-yl)-1-(phenoxy)-2-(4'-chlorphenyl)-propan2-ol,
1-(1',2',4'-Triazol-1'-yl)-1-(4'-chlorphenoxy)-2-(4'-chlorphenyl)propan-2-ol,
1-(1',2',4'-Triazol-1'-yl)-1-(2',4'-dichlorphenoxy)-2-(4'-chlorphenyl)propan-2-ol,
1-(1',2',4'-Triazol-1'-yl)-1-(4'-phenylphenoxy)-2-(4'-chlorphenyl)propan-2-ol,
1-(1',2',4'-Triazol-1'-yl)-1-(phenoxy)-2-(2',4'-dichlorphenyl)propan-2-ol,
1-(1',2',4'-Triazol-1'-yl)-1-(4'-chlorphenoxy)-2-(2',4'-dichlorphenyl)propan-2-ol,
1-(1',2',4'-Triazol-1'-yl)-1-(2',4'-dichlorphenoxy)-2-(2',4'-dichlorphenyl)propan-2-ol,
1-(1',2',4'-Triazol-1'-yl)-1-(4'-phenylphenoxy)-2-(2',4'-dichlorphenyl)propan-2-ol,
1-(1',2',4'-Triazol-1'-yl)-1-(4'-chlorphenoxy)-2-(4'-chlorphenyl)butan-2-ol,
1-(1',2',4'-Triazol-1'-yl)-1-(2',4'-dichlorphenoxy)-2-(4'-chlorphenyl)butan-2-ol,
1-(1',2',4'-Triazol-1'-yl)-1-(4'-chlorphenoxy)-2-(2',4'-dichlorphenyl)butan-2-ol,
1-(1',2',4'-Triazol-1'-yl)-1-(2',4'-dichlorphenoxy)-2-(2',4'-dichlorphenyl)butan-2-ol,
1-(1',2',4'-Triazol-1'-yl)-1-(4'-chlorphenoxy)-2-(4'-chlorphenyl)buten-3-ol-2,
1-(1',2',4'-Triazol-1'-yl)-1-(2',4'-dichlorphenoxy)-2-(4'-chlorphenyl)buten-3-ol-2,
1-(1',2',4'-Triazol-1'-yl)-1-(4'-chlorphenoxy)-2-(2',4'-dichlorphenyl)buten-3-ol-2,
1-(1',2',4'-Triazol-1'-yl)-1-(4'-chlorphenoxy)-2-(4'-chlorbenzyl)-3,3-dimethylbutan-2-ol,
1-(1',2',4'-Triazol-1'-yl)-1-(2',4'-dichlorphenoxy)-2-(4'-chlorbenzyl)-3,3-dimethylbutan-2-ol.

Als Verbindungen der Formel (I) werden beispielsweise genannt:

| Nr. | $R^1$ | n | $R^2$ | $R^3$ | $R^4$ |
|---|---|---|---|---|---|
| 12 | 4-Cl | 1 | tert.-$C_4H_9$ | $CH_2$-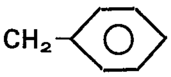 | $CH_2-CH=CH_2$ |
| 13 | 4-Cl | 1 | tert.-$C_4H_9$ | $C\equiv CH$ | $CH_2$-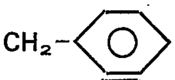-Cl |
| 14 | 4-$C_6H_5$ | 1 | tert.-$C_4H_9$ | $CH_2$-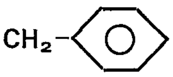 | $CH_2$-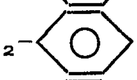-Cl |
| 20 | 4-Cl | 1 | 2,4-$Cl_2-C_6H_3-$ | $CH_3$ | $CH_2$-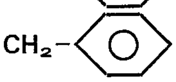-Cl |
| 23 | 4-Cl | 1 | 4-$Cl-C_6H_4-$ | $CH_3$ | $CH_2-CH=CH_2$ |
| 24 | 4-Cl | 1 | 4-$Cl-C_6H_4-$ | $CH_3$ | $CH_2$-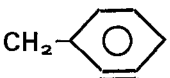 |
| 29 | 4-$C_6H_5$ | 1 | tert.-$C_4H_9$ | $CH=CH_2$ | $CH_2$-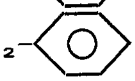 |
| 31 | 4-Cl | 1 | $CH_3$ | $C_2H_5$ | $CH_2-CH=CH_2$ |
| 32 | 4-Cl | 1 | $CH_3$ | iso-$C_3H_7$ | $CH_3$ |
| 33 | 4-Cl | 1 | $CH_3$ | iso-$C_3H_7$ | $CH_2$-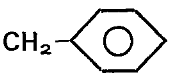 |
| 34 | 4-Cl | 1 | $CH_3$ | n-$C_4H_9$ | $CH_3$ |
| 36 | 4-Cl | 1 | $CH_3$ | $CH=CH_2$ | $CH_3$ |

Es wurde weiter gefunden, dass man die Verbindungen der Formel (I) erhält, wenn man die Carbinole der Formel (II)

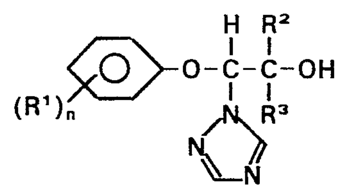

(II)

worin $R^1$, $R^2$, $R^3$ und n die oben angegebenen Bedeutungen haben, oder ihre Alkali- oder quartären Ammoniumsalze mit einem Alkylierungsmittel der Formel (III)

$$L-R^4 \qquad (III)$$

worin $R^4$ die oben angegebene Bedeutung hat und L für eine nucleophil verdrängbare Abgangsgruppe steht, gegebenenfalls in Gegenwart eines Lösungs- oder Verdünnungsmittels, gegebenenfalls in Gegenwart anorganischer oder organischer Basen und gegebenenfalls in Gegenwart eines Reaktionsbeschleunigers bei Temperaturen zwischen 0 und 100°C umsetzt.

Für die oben erwähnte nucleophil verdrängbare Abgangsgruppe L seien beispielsweise genannt: Halogen wie Chlor, Brom oder Jod; Alkylsulfat wie Methylsulfat; gegebenenfalls substituierte Alkylsulfonyloxyreste wie Methansulfonyloxy oder Trifluormethansulfonyloxyreste oder Arylsulfonyloxyreste wie der Methylphenylsulfonyloxyrest.

Geeignete anorganische oder organische Basen, die gegebenenfalls auch als säurebindende Mittel bei der Reaktion eingesetzt werden können, sind beispielsweise Alkali- und Erdalkalihydroxide wie Natriumhydroxid, Kaliumhydroxid, Calciumhydroxid; Alkalicarbonate wie Kalium- oder Natriumcarbonat; Alkalihydride wie Natriumhydrid; Alkali- oder Erdalkalialkoholate wie Natriummethylat, Magnesiummethylat oder Natriumisopropylat; tertiäre Amine wie Trimethylamin, Triäthylamin, N,N-Dimethylanilin, N,N-Dimethylcyclohexylamin, N-Methylpiperidin oder Pyridin. Es können aber auch andere übliche Basen verwendet werden.

Mit geeigneten Basen wie zum Beispiel Alkalihydrid wie Natriumhydrid oder Lithiumalkyl wie Butyllithium oder mit Alkali- oder Erdalkalialkoholaten wie Natriummethylat können die Alkohole der Formel (II) auch vor der Umsetzung zunächst in ihre Alkoholatsalze übergeführt und danach zur Reaktion gebracht werden.

Zu den Lösungs- bzw. Verdünnungsmitteln gehören z.B. Halogenkohlenwasserstoffe wie beispielsweise Methylenchlorid, Chloroform, 1,2-Dichlorethan, Chlorbenzol; aliphatische oder aromatische Kohlenwasserstoffe wie Cyclohexan, Petroläther, Benzol, Toluol oder Xylole; Ester wie Essigsäureäthylester; Amide wie Dimethylformamid; Nitrile wie Acetonitril; Sulfoxide wie Dimethylsulfoxid; Ketone wie Aceton oder Methylethylketon; Äther wie Diethyläther, Tetrahydrofuran oder Dioxan oder entsprechende Gemische.

Als Reaktionsbeschleuniger kommen beispielsweise Metallhalogenide z.B. Kaliumjodid; Kronenäther; quartäre Ammoniumverbindungen z.B. Tetrabutylammoniumjodid oder Säuren oder Mischungen dieser Reaktionsbeschleuniger in Frage. Die erfindungsgemässen Umsetzungen werden im allgemeinen bei Temperaturen zwischen 0 und 150°C in einem Zeitraum von 1 bis 60 h, drucklos oder unter Druck, kontinuierlich oder diskontinuierlich durchgeführt.

Zur Abtrennung der erfindungsgemässen Verbindungen von den Reaktionsmischungen wird nach üblichen Methoden vorgegangen. Im allgemeinen bedürfen die so erhaltenen Produkte keiner weiteren Reinigung, sie können aber nach bekannten Methoden wie Umkristallisation, Extraktion, Destillation oder Chromatographie weiter gereinigt werden.

Falls gewünscht, können die Carbinoläther der Formel (I) nach bekannten Verfahren in ihre für Pflanzen verträglichen Salze oder in ihre Metallkomplexverbindungen übergeführt werden. Als Salze seien beispielsweise die Hydrochloride, -bromide, -sulfate, -nitrate, -phosphate, -oxalate oder -dodecylbenzolsulfonate genannt.

Für die Metallkomplexverbindungen seien die Komplexverbindungen der Carbinoläther mit Schwermetallsalzen wie beispielsweise mit Zinkchlorid, Kupfer-II-chlorid, Kupfer-II-sulfat, Mangan-II-chlorid, Eisen-II-chlorid oder Nickel-II-bromid genannt.

Die neuen Verbindungen und ihre Salze und Metallkomplexverbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen.

Unter Kulturpflanzen sind in diesem Zusammenhang insbesondere Weizen, Roggen, Gerste, Hafer, Reis, Mais, Baumwolle, Soja, Kaffee, Zuckerrohr, Obst und Zierpflanzen im Gartenbau, sowie Gemüse wie Gurken, Bohnen und Kürbisgewächse zu verstehen.

Die neuen Verbindungen sind insbesondere geeignet zur Bekämpfung folgender Pflanzenkrankheiten:

*Erysiphe graminis* (echter Mehltau) in Getreide,
*Erysiphe cichoriacearum* (echter Mehltau) an Kürbisgewächsen,
*Podosphaera leucotricha* an Äpfeln,
*Uncinula necator* an Reben,
*Erysiphe polygoni* an Bohnen,
*Sphaerotheca pannosa* an Rosen,
*Puccinia*-Arten an Getreide,
*Rhizoctonia solani* an Baumwolle sowie
*Helminthosporium*-Arten an Getreide,
*Ustilago*-Arten an Getreide und Zuckerrohr,
*Rhynchosporium secale* an Getreide,
*Venturia inaequalis* (Apfelschorf),
*Mycosphaerella musicola* an Bananen,
*Piricularia oryzae* an Reis,
*Pellicularia sasakii* an Reis,
*Botrytis cinerea* an Weinreben und Erdbeeren.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die erfindungsgemässen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmässige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Äthanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyäthylen — Fettalkohol — Äther, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95 Gew.% Wirkstoff, vorzugsweise zwischen 0,5 und 90.

Die Aufwandmengen liegen je nach Art des gewünschten Effektes zwischen 0,1 und 3 kg Wirkstoff oder mehr je ha.

Die neuen Wirkstoffe können auch im Materialschutz u.a. zur Bekämpfung holzzerstörender Pilze wie *Coniophora puteana* und *Polystictus venicolor* verwendet werden. Die Anwendung erfolgt in der Weise, dass man das Holz mit diesen Mitteln behandelt, beispielsweise tränkt oder anstreicht.

Die Mittel bzw. die daraus hergestellten gebrauchsfertigen Zubereitungen wie Lösungen,

Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Giessen.

Die erfindungsgemässen Mittel können in den verschiedenen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrösserung des fungiziden Wirkungsspektrums.

*I. Herstellungsbeispiele*

Die Herstellung der neuen Verbindungen der Formel (I) wird durch folgende Beispiele erläutert:

*Beispiel 1*

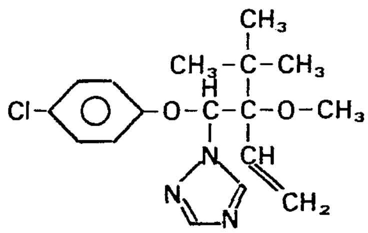

In 80 ml absolut trockenem Tetrahydrofuran (THF) werden unter Stickstoff 1,25 g NaH vorgelegt und unter Rühren mit einer Lösung von 12,8 g 1-(1',2',4'-Triazol-1'-yl)-1-(4'-chlorphenoxy)-2-vinyl-3,3-dimethylbutan-2-ol in 80 ml abs. THF versetzt. Nach 3stündigem Rühren bei Raumtemperatur (20°C) werden 6,8 g Methyljodid zugetropft und 12 h gerührt. Das überschüssige NaH wird danach mit Wasser zersetzt. Nach Zugabe von 300 ml Äther wird die organische Phase zweimal mit Wasser gewaschen, getrocknet und eingeengt. Aus den verbleibenden 13,4 g Öl kristallisieren nach Verreiben in Petroläther 9,7 g 1-(1',2',4'-Triazol-1'-yl)-1-(4'-chlorphenoxy)-2-vinyl-3,3-dimethylbutyl-2-methyläther vom Schmelzpunkt 96 bis 98°C. (Nr. 1)

| | | | | |
|---|---|---|---|---|
| berechnet: | C 60,8 | H 6,6 | N 12,5 | Cl 10,6 |
| gefunden: | C 60,7 | H 6,5 | N 12,8 | Cl 10,5 |

Das als Ausgangsmaterial verwendete 1-(1',2',4'-Triazol-1'-yl)-1-(4'-chlorphenoxy)-2-vinyl-3,3-dimethylbutan-2-ol wurde wie folgt hergestellt:

Zu einer Lösung von 0,22 mol Vinylmagnesiumchlorid in 140 ml abs. THF wird unter Rühren eine Lösung von 29,4 g (0,1 mol) 1-(1',2',4'-Triazol-1'-yl)-1-(4'-chlorphenoxy)-3,3-dimethylbutan-2-ol in 100 ml abs. THF bei 20 bis 35°C zugetropft. Anschliessend wird für 5 h bei 40 bis 45°C gerührt. Unter Kühlung wird mit 150 ml Wasser zersetzt und der ausgefallene Niederschlag in 200 ml 20%iger wässriger Ammoniumchloridlösung gelöst. Die organische Phase wird abgetrennt und zweimal mit Wasser gewaschen, getrocknet und eingeengt. Das verbleibende Öl wird aus Cyclohexan mit wenig Essigesterzusatz umkristallisiert. So erhält man 10 g 1-(1',2',4'-Triazol-1'-yl)-1-(4'-chlorphenoxy)-2-vinyl-3,3-dimethylbutan-2-ol vom Schmelzpunkt 152 bis 153°C.

| | | | | |
|---|---|---|---|---|
| berechnet: | C 59,7 | H 6,3 | N 13,1 | Cl 11,0 |
| gefunden: | C 59,8 | H 6,1 | N 13,3 | Cl 10,8 |

*Beispiel 2*

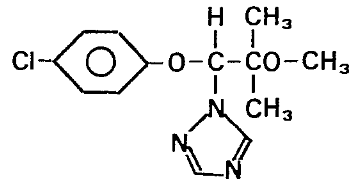

Zu einer Suspension von 4 g Natriumhydrid in 100 ml THF wird bei Raumtemperatur (20°C) eine Lösung von 21,4 g 1-(1',2',4'-Triazol-1'-yl)-1-(4'-chlorphenoxy)-2-methylpropanol-2 in 200 ml THF getropft und dann 4 h lang nachgerührt. Nach der Zugabe von 15,9 g Methyljodid wird über Nacht (12 h) nachgerührt. Das überschüssige Natriumhydrid wird mit Wasser zersetzt, das Reaktionsgemisch anschliessend mit Schwefelsäure neutralisiert und eingeengt. Der Rückstand wird in 500 ml Methylenchlorid gelöst und zweimal mit Wasser gewaschen. Die organische Phase wird abgetrennt, getrocknet und eingeengt. Man erhält so 18 g 1-(1',2',4'-Triazol-1'-yl)-1-(4'-chlorphenoxy)-2-methylpropyl-2-methyläther (Nr. 2) als Öl ($n_D^{20}$ = 1,5251)

| | | | | |
|---|---|---|---|---|
| berechnet: | C 55,4 | H 5,7 | N 14,9 | Cl 12,5 |
| gefunden: | C 55,2 | H 5,6 | N 14,5 | Cl 12,5 |

Das 1-(1',2',4'-Triazol-1'-yl)-1-(4'-chlorphenoxy)-2-methylpropanol-2 wird wie folgt hergestellt:

Zu einer Lösung von 1 mol Methylmagnesiumjodid in 100 ml Diethyläther (hergestellt aus 24 g Magnesiumspänen und 142 g Methyljodid) wird unter Rühren eine Lösung von 56,3 g 2-(1',2',4'-Triazol-1'-yl)-2-(4'-chlorphenoxy)essigsäuremethylester in 100 ml Diethyläther langsam getropft. Nach einstündigem Rühren bei 40°C wird über Nacht stehen gelassen. Nach Zugabe von 150 ml gesättigter Ammoniumchloridlösung wird die Ätherphase abgetrennt, die wässrige Phase noch zweimal ausgeäthert und die vereinigte organische Phase getrocknet und eingeengt. Aus Diisopropyläther kristallisieren 22 g 1-(1',2',4'-Triazol-1'-yl)-1-(4'-chlorphenoxy)-2-methylpropanol-2 vom Schmelzpunkt 99 bis 100°C.

Entsprechend wurden folgende Substanzen der allgemeinen Formel (I) hergestellt und durch UR- und NMR-Spektroskopie sowie durch eine Elementaranalyse charakterisiert.

| Nr. | $R^1$ | n | $R^2$ | $R^3$ | $R^4$ | Fp [°C] |
|---|---|---|---|---|---|---|
| 3 | 4-Cl | 1 | tert.-$C_4H_9$ | $CH_3$ | $CH_3$ | Öl |
| 4 | 4-Cl | 1 | $CH_3$ | tert.-$C_4H_9$ | $CH_2-CH=CH_2$ | Öl |
| 5 | 4-Cl | 1 | tert.-$C_4H_9$ | $CH=CH_2$ | $CH_2-CH=CH_2$ | Öl |
| 6 | 4-Cl | 1 | tert.-$C_4H_9$ | $CH_3$ | 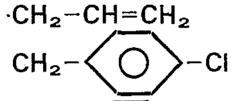 | Öl |
| 7 | 4-Cl | 1 | tert.-$C_4H_9$ | $CH=CH_2$ | 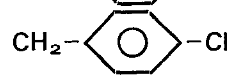 | Öl |
| 8 | 4-$C_6H_5$ | 1 | $CH_3$ | $CH_3$ | $CH_3$ | 86-89 |
| 9 | 4-Cl | 1 | tert.-$C_4H_9$ | $C\equiv CH$ | $CH_3$ | 116-118 |
| 10 | 4-Cl | 1 | tert.-$C_4H_9$ | 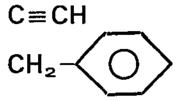 | $CH_3$ | Öl |
| 11 | 4-Cl | 1 | tert.-$C_4H_9$ | $C\equiv CH$ | $CH_2-CH=CH_2$ | Öl |
| 15 | 4-$C_6H_5$ | 1 | tert.-$C_4H_9$ | $CH_3$ | $CH_3$ | Öl |
| 16 | 4-$C_6H_5$ | 1 | tert.-$C_4H_9$ | $CH_3$ | $CH_2-CH=CH_2$ | Öl |
| 17 | 4-$C_6H_5$ | 1 | tert.-$C_4H_9$ | $CH_3$ | 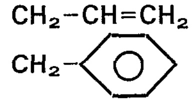 | Öl |
| 18 | 4-Cl | 1 | 2,4-$Cl_2-C_6H_3-$ | $CH_3$ | $CH_3$ | Öl |
| 19 | 4-Cl | 1 | 2,4-$Cl_2-C_6H_3-$ | $CH_3$ | $CH_2-CH=CH_2$ | Öl |
| 21 | 4-$C_6H_5$ | 1 | $CH_3$ | $CH_3$ | n-$C_4H_9$ | |
| 22 | 4-Cl | 1 | 4-Cl-$C_6H_4-$ | $CH_3$ | $CH_3$ | 98-100 |
| 25 | 4-Cl | 1 | tert.-$C_4H_9$ | 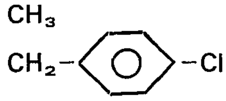 | $CH_3$ | Öl |
| 26 | 4-$C_6H_5$ | 1 | tert.-$C_4H_9$ | $CH=CH_2$ | $CH_3$ | Öl |
| 27 | 4-$C_6H_5$ | 1 | tert.-$C_4H_9$ | $CH=CH_2$ | $C_2H_5$ | |
| 28 | 4-$C_6H_5$ | 1 | tert.-$C_4H_9$ | $CH=CH_2$ | $CH_2-CH=CH_2$ | Öl |
| 30 | 4-Cl | 1 | $C_2H_5$ | $C_2H_5$ | $CH_3$ | Öl |
| 35 | 4-Cl | 1 | $CH_3$ | $CH_3$ | 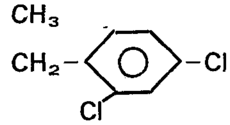 | Öl |
| 37 | 4-Cl | 1 | $CH_3$ | $CH=CH_2$ | $CH_2-CH=CH_2$ | |
| 38 | 4-$C_6H_5$ | 1 | $CH_3$ | $CH_3$ | 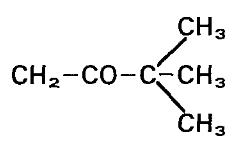 | Öl |
| 39 | 2,4-$Cl_2$ | 2 | 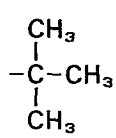 | $CH_3$ | $CH_3$ | 103-105 |

*II. Zubereitungen*

Beispiele für Zubereitungen sind:

I. Man vermischt 90 Gewichtsteile der Verbindung des Beispiels 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung des Beispiels 1 werden in einer Mischung gelöst, die aus 80 Gewichtsteilen Xylol, 10 Gewichtsteilen des Anlagerungsproduktes von 8 bis 10 mol Äthylenoxid an 1 mol Ölsäure-N-monoäthanolamid, 5 Gewichtsteilen Calciumsalz der Dodecylbenzolsulfonsäure und 5 Gewichststeilen des Anlagerungsproduktes von 40 mol Äthylenoxid an 1 mol

Ricinusöl besteht. Durch Ausgiessen und feines Verteilen der Lösung in 100 000 Gewichtsteilen Wasser erhält man eine wässrige Dispersion, die 0,02 Gew.% des Wirkstoffs enthält.

III. 20 Gewichtsteile der Verbindung des Beispiel 1 werden in einer Mischung gelöst, die aus 40 Gewichtsteilen Cyclohexanon, 30 Gewichtsteilen Isobutanol, 20 Gewichtsteilen des Anlagerungsproduktes von 40 mol Äthylenoxid an 1 mol Ricinusöl besteht. Durch Eingiessen und feines Verteilen der Lösung in 100 000 Gewichtsteilen Wasser erhält man eine wässrige Dispersion, die 0,02 Gew.% des Wirkstoffs enthält.

IV. 20 Gewichtsteile der Verbindung des Beispiels 1 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 mol Äthylenoxid an 1 mol Ricinusöl besteht. Durch Eingiessen und feines Verteilen der Lösung in 100 000 Gewichtsteilen Wasser erhält man eine wässrige Dispersion, die 0,02 Gew.% des Wirkstoffs enthält.

V. 20 Gewichtsteile der Verbindung des Beispiels 1 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile der Verbindung des Beispiels 1 werden mit 97 Gewichtsteilen feinteiligem Kaolin innig vermischt. Man erhält auf diese Weise ein Stäubemittel, das 3 Gew.% des Wirkstoffs enthält.

VII. 30 Gewichtsteile der Verbindung des Beispiels 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung des Beispiels 1 werden mit 10 Teilen Natriumsalz eines Phenolsulfonsäureharnstoffformaldehydkondensates, 2 Teilen Kieselgel und 48 Teilen Wasser innig vermischt. Man erhält eine stabile wässrige Dispersion. Durch Verdünnen mit 100 000 Gewichtsteilen Wasser erhält man eine wässrige Dispersion, die 0,04 Gew.% Wirkstoff enthält.

IX. 20 Teile der Verbindung des Beispiels 1 werden mit 2 Teilen Calciumsalz der Dodecylbenzolsulfonsäure, 8 Teilen Fettalkoholpolyglykoläther, 2 Teilen Natriumsalz eines Phenolsulfonsäureharnstoffformaldehydkondensates und 68 Teilen eines paraffinischen Mineralöls innig vermischt. Man erhält eine stabile ölige Dispersion.

*III. Biologische Versuche*

Für die folgenden biologischen Versuche wurde folgender bekannter Vergleichswirkstoff verwendet:

1-[2'-(2'',4''-Dichlorphenyl)-2'-(2''-propenyloxy)]äthyl-1H-imidazol (Verbindung A), bekannt aus GB-C Nr. 1318590.

*Versuch 1*

Wirkung gegen Weizenmehltau

Blätter von in Töpfen gewachsenen Weizenkeimlingen der Sorte «Jubilar» werden mit wässrigen Emulsionen aus 80% (Gewichtsprozent) Wirkstoff und 20% Emulgiermittel besprüht und nach dem Antrocknen des Spritzbelages mit Oidien (Sporen) des Weizenmehltaus *(Erysiphe graminis var. tritici)* bestäubt. Die Versuchspflanzen werden anschliessend im Gewächshaus bei Temperaturen zwischen 20 und 22°C und 75 bis 80% relativer Luftfeuchtigkeit aufgestellt. Nach 10 Tagen wird das Ausmass der Mehltauentwicklung ermittelt.

| Wirkstoff Nr. | Befall der Blätter nach Spritzung mit %iger Wirkstoffbrühe | | |
|---|---|---|---|
| | 0,025 | 0,012 | 0,006 |
| 1 | 0 | 0 | 0 |
| 3 | 0 | 0 | 0 |
| 4 | 0 | 0 | 0 |
| 5 | 0 | 0 | 0 |
| 6 | 0 | 0 | 0 |
| 7 | 0 | 0 | 0 |
| 8 | 0 | 0 | 0 |
| 9 | 0 | 0 | 0 |
| 12 | 0 | 0 | 0 |
| 13 | 0 | 0 | 0 |
| 15 | 0 | 0 | 0 |
| 16 | 0 | 0 | 0 |
| 17 | 0 | 0 | 1 |
| 18 | 0 | 0 | 0 |
| 19 | 0 | 0 | 0 |
| 22 | 0 | 0 | 0 |
| 25 | 0 | 0 | 0 |
| 30 | 0 | 0 | 0 |
| 35 | 0 | 0 | 1 |
| 38 | 0 | 0 | 1 |
| A (bekannt) | 3 | 3 | 4 |
| Kontrolle (unbehandelt) | | 5 | |

0 = kein Pilzbefall, abgestuft bis 5 = Totalbefall

*Versuch 2*

Wirkung gegen Weizenbraunrost

Blätter von in Töpfen gewachsenen Weizensämlingen der Sorte «Caribo» werden mit Sporen des Braunrostes *(Puccinia recondita)* bestäubt. Danach werden die Töpfe für 24 h bei 20 bis 22°C in eine Kammer mit hoher Luftfeuchtigkeit (90 bis 95%) gestellt. Während dieser Zeit keimen die Sporen aus und die Keumschläuche dringen in das Blattgewebe ein. Die infizierten Pflanzen werden anschliessend mit 0,025, 0,012 und 0,006%igen (Gew.%) wässrigen Spritzbrühen, die 80% Wirkstoff und 20% Ligninsulfonat in der Trockensubstanz enthalten, tropfnass gespritzt. Nach dem Antrocknen des Spritzbelages werden die Versuchspflanzen im Gewächshaus bei Temperaturen zwischen 20 und 22°C und 65 bis 70% relativer Luftfeuchte aufgestellt. Nach 8 Tagen wird das Ausmass der Rostpilzentwicklung auf den Blättern ermittelt.

| Wirkstoff Nr. | Befall der Blätter nach Spritzung mit %iger Wirkstoffbrühe | | |
|---|---|---|---|
| | 0,025 | 0,012 | 0,006 |
| 1 | 0 | 0 | 1 |
| 3 | 0 | 0 | 0 |
| 4 | 0 | 0 | 0 |
| 6 | 0 | 1 | 2 |
| 8 | 0 | 0 | 1 |
| 9 | 0 | 1 | 2-3 |
| 15 | 0 | 0 | 0 |
| 16 | 0 | 1 | 2 |
| 18 | 1 | 1 | 3 |
| 26 | 0 | 0 | – |
| 28 | 0 | 0 | – |
| 30 | 0 | 1 | 3 |
| 38 | 1 | 2 | 3 |
| A (bekannt) | 4 | 5 | 5 |
| Kontrolle (unbehandelt) | | 5 | |

0 = kein Pilzbefall, abgestuft bis 5 = Totalbefall

**Patentansprüche für die Vertragsstaaten: BE, CH, DE, FR, GB, IT, LI, LU, NL, SE**

1. Carbinoläther der allgemeinen Formel (I)

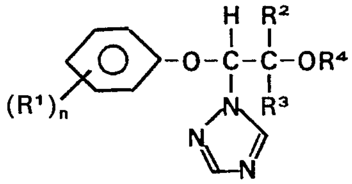
(I)

in welcher:

$R^1$ Halogen oder Phenyl

$R^2$ Alkyl mit 1 bis 6 C-Atomen oder gegebenenfalls durch Chlor substituiertes Phenyl

$R^3$ Alkyl mit 1 bis 6 C-Atomen, Vinyl, Äthinyl oder gegebenenfalls durch Chlor substituiertes Benzyl

$R^4$ Alkyl mit 1 bis 4 C-Atomen, den Allylrest gegebenenfalls durch Chlor substituiertes Benzyl oder 3,3-Dimethylbutan-2-on-1-yl und

n 0, 1 oder 2 bedeutet

sowie deren Salze und Metallkomplexverbindungen.

2. Carbinoläther gemäss Anspruch 1, ausgewählt aus der Gruppe, bestehend aus
[1-(1′,2′,4′-Triazol-1′-yl)-(4′-chlorphenoxy)-2,3,3-trimethylbutyl-2]-methyläther,
[1-(1′,2′,4′-Triazol-1′-yl)-1-(4′-chlorphenoxy)-2,3,3-trimethylbutyl-2]-allyläther und
[1-(1′,2′,4′-Triazol-1′-yl)-1-(4′-chlorphenoxy)-2-vinyl-3,3-dimethylbutyl-2]-methyläther.

3. Verfahren zur Herstellung eines Carbinoläthers gemäss Anspruch 1, dadurch gekennzeichnet, dass man entweder

a) ein Carbinol der Formel (II)

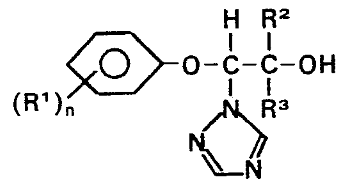
(II)

worin $R^1$, $R^2$, $R^3$ und n die im Anspruch 1 angegebenen Bedeutungen haben, oder deren Alkali- oder quartären Ammoniumsalze mit einem Alkylierungsmittel der Formel (III)

$$L\text{-}R^4 \qquad (III)$$

worin $R^4$ die oben erläuterte Bedeutung hat und L für eine nucleophil verdrängbare Abgangsgruppe steht, gegebenenfalls in Gegenwart eines Lösungs- oder Verdünnungsmittels, gegebenenfalls in Gegenwart anorganischer oder organischer Basen und gegebenenfalls in Gegenwart eines Reaktionsbeschleunigers bei Temperaturen zwischen 0 und 100°C umsetzt oder

b) im Falle der Herstellung eines Carbinoläthers nach Anspruch 1 mit $R_2=R_3=R_5$ wobei diese Reste jeweils Alkylreste mit 1 bis 6 C-Atomen bedeuten zunächst aus einem Ester der Formel (V)

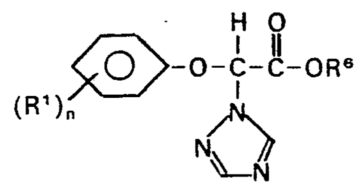
(V)

worin $R^1$ und n die oben angegebene Bedeutung haben und $R^6$ für Alkyl mit 1 bis 4 C-Atomen steht, mit Grignard-Reagentien der Formel (VI)

$$R^5\text{-}Mg\text{-}Hal \qquad (VI)$$

worin $R^5$ die oben angegebene Bedeutung hat und Hal für Chlor, Brom oder Jod steht, in Gegenwart inerter Lösungsmittel umsetzt und das daraus

erhältliche Carbinol entsprechend Verfahren a) alkyliert.

4. Fungizid, enthaltend einen Carbinoläther gemäss Anspruch 1 oder Anspruch 2.

5. Fungizid, enthaltend einen festen oder flüssigen Trägerstoff und einen Carbinoläther gemäss Anspruch 1 oder Anspruch 2.

6. Verfahren zur Herstellung eines Fungizids, dadurch gekennzeichnet, dass man einen festen oder flüssigen Trägerstoff mit einem Carbinoläther gemäss Anspruch 1 vermischt.

7. Verfahren zur Bekämpfung von Pilzen, dadurch gekennzeichnet, dass man die Pilze oder die vor Pilzbefall zu schützenden Gegenstände mit einem Carbinoläther gemäss Anspruch 1 behandelt.

**Patentansprüche für den Vertragsstaat: AT**

1. Fungizid enthaltend einen Carbinoläther der Formel (I)

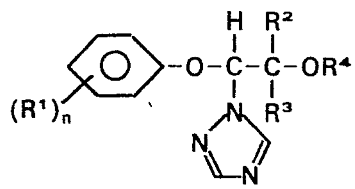

(I)

in welcher

$R^1$ Halogen oder Phenyl

$R^2$ Alkyl mit 1 bis 6 C-Atomen oder gegebenenfalls durch Chlor substituiertes Phenyl

$R^3$ Alkyl mit 1 bis 6 C-Atomen, Vinyl, Äthinyl, oder gegebenenfalls durch Chlor substituiertes Benzyl

$R^4$ Alkyl mit 1 bis 4 C-Atomen, den Allylrest, gegebenenfalls durch Chlor substituiertes Benzyl oder 3,3-Dimethylbutan-2-on-1-yl und

n 0, 1 oder 2 bedeutet

sowie deren Salze und Metallkomplexverbindungen.

2. Fungizid gemäss Anspruch 1, enthaltend einen Carbinoläther, ausgewählt aus der Gruppe, bestehend aus

[1-(1',2',4'-Triazol-1'-yl)-1-(4'-chlorphenoxy)-2,3,3-trimethylbutyl-2]-methyläther,

[1-(1',2',4'-Triazol-1'-yl)-1-(4'-chlorphenoxy)-2,3,3-trimethylbutyl-2]-allyläther und

[1-(1',2',4'-Triazol-1'-yl)-1-(4'-chlorphenoxy)-2-vinyl-3,3-dimethylbutyl-2]-methyläther.

3. Verfahren zur Herstellung eines Carbinoläthers gemäss Anspruch 1, dadurch gekennzeichnet, dass man entweder

a) ein Carbinol der Formel (II)

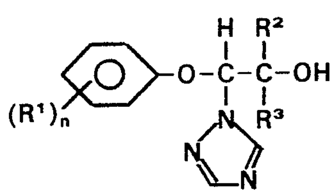

(II)

worin $R^1$, $R^2$, $R^3$ und n die im Anspruch 1 angegebenen Bedeutungen haben, oder deren Alkali- oder quartären Ammoniumsalze mit einem Alkylierungsmittel der Formel (III)

$$L-R^4 \qquad (III)$$

worin $R^4$ die oben erläuterte Bedeutung hat und L für eine nucleophil verdrängbare Abgangsgruppe steht, gegebenenfalls in Gegenwart eines Lösungs- oder Verdünnungsmittels, gegebenenfalls in Gegenwart anorganischer oder organischer Basen und gegebenenfalls in Gegenwart eines Reaktionsbeschleunigers bei Temperaturen zwischen 0 und 100°C umsetzt oder

b) im Falle der Herstellung eines Carbinoläthers nach Anspruch 1 mit $R_2=R_3=R_5$ wobei diese Reste jeweils Alkylreste mit 1 bis 6 C-Atomen bedeuten zunächst aus einem Ester der Formel (V)

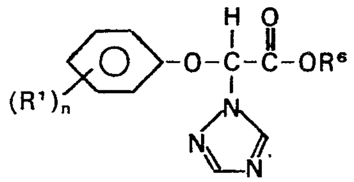

(V)

worin $R^1$ und n die oben angegebene Bedeutung haben und $R^6$ für Alkyl mit 1 bis 4 C-Atomen steht, mit Grignard-Reagentien der Formel (VI)

$$R^5-Mg-Hal \qquad (VI)$$

worin $R^5$ die oben angegebene Bedeutung hat und Hal für Chlor, Brom oder Jod steht, in Gegenwart inerter Lösungsmittel umsetzt und das daraus erhältliche Carbinol entsprechend Verfahren a) alkyliert.

4. Fungizid, enthaltend einen festen oder flüssigen Trägerstoff und einen Carbinoläther wie in Anspruch 1 oder 2 definiert.

5. Verfahren zur Herstellung eines Fungizids, dadurch gekennzeichnet, dass man einen festen oder flüssigen Trägerstoff mit einem Carbinoläther wie in Anspruch 1 definiert vermischt.

6. Verfahren zur Bekämpfung von Pilzen, dadurch gekennzeichnet, dass man die Pilze oder die vor Pilzbefall zu schützenden Gegenstände mit einem Carbinoläther wie in Anspruch 1 definiert behandelt.

**Claims for the contracting states: BE, CH, LI, DE, FR, GB, IT, LU, NL, SE**

1. Carbinol ethers of the general formula (I)

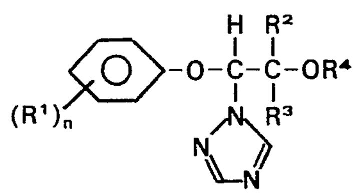

(I)

where $R^1$ denotes halogen or phenyl, $R^2$ denotes alkyl of 1 to 6 carbon atoms or unsubstituted or chlorine-substituted phenyl, $R^3$ denotes alkyl of 1 to 6 carbon atoms, vinyl, ethynyl or unsubstituted

or chlorine-substituted benzyl, $R^4$ denotes alkyl of 1 to 4 carbon atoms, allyl, unsubstituted or chlorine-substituted benzyl, or 3,3-dimethyl-butan-2-on-1-yl, and n denotes 0, 1 or 2, and salts and metal complex compounds thereof.

2. A carbinolether as claimed in claim 1, selected from the group consisting of
[1-(1',2',4'-triazol-1'-yl)-1-(4'-chlorophenoxy)-2,3,3-trimethylbut-2-yl]methylether,
[1-(1',2',4'-triazol-1'-yl)-1-(4'-chlorophenoxy)-2,3,3-trimethylbut-2-yl]allylether and
[1-(1',2',4'-triazol-1'-yl)-1-(4'-chlorophenoxy)-2-vinyl-3,3-dimethylbut-2-yl]methylether.

3. A process for the manufacture of a carbinolether as claimed in claim 1, characterized in that either

a) a carbinol of the formula (II)

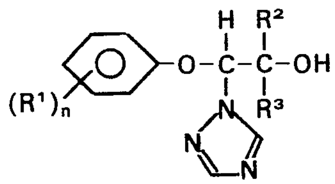
(II)

where $R^1$, $R^2$, $R^3$ and n have the meanings given in claim 1, or an alkalimetal or quaternary ammoniumsalt thereof, is reacted at from 0 to 100°C with an alkylating agent of the formula (III)

$$L-R^4 \quad \text{(III)}$$

where $R^4$ has the above meanings and L denotes a nucleophilically displaceable leaving group, in the presence or absence of a solvent or diluent, in the presence or absence of an inorganic or organic base, and in the presence or absence of a reaction accelerator, or

b) in the case of the manufacture of a carbinolether as claimed in claim 1, where $R^2=R^3=R^5$, these radicals each denoting alkyl of 1 to 6 carbon atoms, a carbinol is first prepared by reacting an ester of the formula (V)

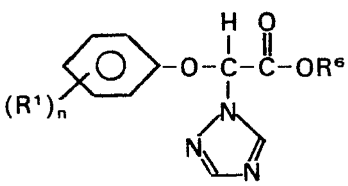
(V)

where $R^1$ and n have the above meanings and $R^6$ denotes alkyl of 1 to 4 carbon atoms, with a Grignard reagent of the fomula (VI)

$$R^5-Mg-Hal \quad \text{(VI)}$$

where $R^5$ has the above meanings and Hal denotes chlorine, bromine or iodine, in the presence of an inert solvent, and is then alkylated according to process a).

4. A fungicide containing a carbinolether as claimed in claim 1 or claim 2.

5. A fungicide containing a solid or liquid carrier and a carbinolether as claimed in claim 1 or claim 2.

6. A process for producing a fungicide, characterized in that a solid or liquid carrier is mixed with a carbinolether as claimed in claim 1.

7. A process for combating fungi, characterized in that the fungi or the objects to be protected against fungus attack are treated with a carbinolether as claimed in claim 1.

**Claims for the contracting state: AT**

1. A fungicide containing a carbinolether of the general formula (I)

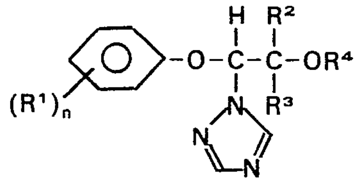
(I)

where $R^1$ denotes halogen or phenyl, $R^2$ denotes alkyl of 1 to 6 carbon atoms or unsubstituted or chlorine-substituted phenyl, $R^3$ denotes alkyl of 1 to 6 carbon atoms, vinyl, ethynyl or unsubstituted or chlorine-substituted benzyl, $R^4$ denotes alkyl of 1 to 4 carbon atoms, allyl, unsubstituted or chlorine-substituted benzyl, or 3,3-dimethyl-butan-2-on-1-yl, and n denotes 0, 1 or 2, and salts and metal complex compounds thereof.

2. A fungicide as claimed in claim 1 containing a carbinolether selected from the group consisting of
[1-(1',2',4'-triazol-1'-yl)-1-(4'-chlorophenoxy)-2,3,3-trimethylbut-2-yl]methylether,
[1-(1',2',4'-triazol-1'-yl)-1-(4'-chlorophenoxy)-2,3,3-trimethylbut-2-yl]allylether and
[1-(1',2',4'-triazol-1'-yl)-1-(4'-chlorophenoxy)-2-vinyl-3,3-dimethylbut-2-yl]methylether.

3. A process for the manufacture of a carbinolether as claimed in claim 1, characterized in that either

a) a carbinol of the formula (II)

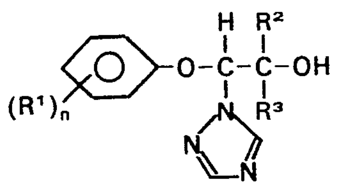
(II)

where $R^1$, $R^2$, $R^3$ and n have the meanings given in claim 1, or an alkalimetal or quaternary ammoniumsalt thereof, is reacted at from 0 to 100°C with an alkylating agent of the formula (III)

$$L-R^4 \quad \text{(III)}$$

where $R^4$ has the above meanings and L denotes a nucleophilically displaceable leaving group, in the presence or absence of a solvent or diluent, in the presence or absence of an inorganic or organic base, and in the presence or absence of a reaction accelerator, or

b) in the case of the manufacture of a carbinolether as claimed in claim 1, where $R^2=R^3=R^5$, these radicals each denoting alkyl of

1 to 6 carbon atoms, a carbinol is first prepared by reacting an ester of the formula (V)

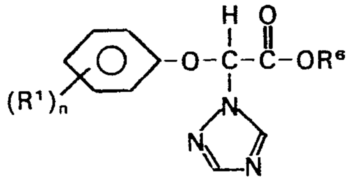 (V)

where $R^1$ and n have the above meanings and $R^6$ denotes alkyl of 1 to 4 carbon atoms, with a Grignard reagent of the formula (VI)

$$R^5-Mg-Hal \quad (VI)$$

where $R^5$ has the above meanings and Hal denotes chlorine, bromine or iodine, in the presence of an inert solvent, and is then alkylated according to process a).

4. A fungicide containing a solid or liquid carrier and a carbinolether as defined in claim 1.

5. A process for producing a fungicide, characterized in that a solid or liquid carrier is mixed with a carbinolether as defined in claim 1.

6. A process for combating fungi, characterized in that the fungi or the objects to be protected against fungus attack are treated with a carbinolether as defined in claim 1.

**Revendications pour les Etats contractants: BE, CH, LI, DE, FR, GB, IT, LU, NL, SE**

1. Esthers de carbinols de la formule générale (I)

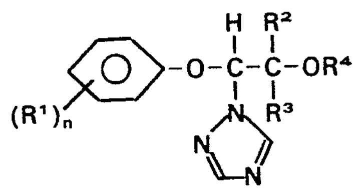 (I)

dans laquelle

$R^1$ désigne un atome d'halogène ou un groupe phényle,

$R^2$ un radical alcoyle en $C_1$ à $C_6$ ou un groupe phényle éventuellement substitué par du chlore,

$R^3$ un radical alcoyle en $C_1$ à $C_6$ ou un groupe vinyle, éthynyle ou benzyle éventuellement substitué par du chlore,

$R^4$ un radical alcoyle en $C_1$ à $C_4$, un groupe allyle, un groupe benzyle éventuellement substitué par du chlore ou le groupe 3,3-diméthylbutane-2-one-1-yle et

n vaut 0, 1 ou 2,

ainsi que leurs sels et complexes avec des métaux.

2. Ether de carbinol suivant la revendication 1, choisi dans le groupe formé par:
l'éther [1-(1',2',4'-triazol-1'-yl)-1-(4'-chlorophénoxy)-2,3,3-triméthylbutyl-2]-méthylique,
l'éther [1-(1',2',4'-triazol-1'-yl)-1-(4'-chlorophénoxy)-2,3,3-triméthylbutyl-2]-allylique, et
l'éther [1-(1',2',4'-triazol-1'-yl)-1-(4'-chlorophénoxy)-2-vinyl-3,3-diméthylbutyl-2]-méthylique.

3. Procédé de préparation d'un éther de carbinol suivant la revendication 1, caractérisé en ce que:

a) on fait réagir un carbinol de la formule (II)

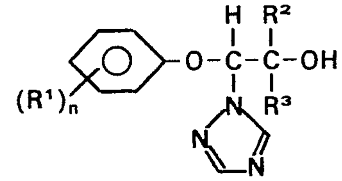 (II)

dans laquelle $R^1$, $R^2$, $R^3$ et n possèdent les significations définies dans la revendication 1, ou un sel de métal alcalin ou d'ammonium d'un carbinol, avec un agent d'alcoylation de la formule (III)

$$L-R^4 \quad (III)$$

dans laquelle $R^4$ possède la signification définie plus haut et L représente un groupe pouvant être éliminé par un clivage nucléophile, à des températures entre 0 et 100°C, le cas échéant dans un solvant ou diluant et éventuellement en présence d'une base inorganique ou organique et/ou en présence d'un accélérateur de la réaction, ou

b) dans le cas de la préparation d'un éther de carbinol suivant la revendication 1 avec $R^2=R^3=R^5=$ un radical alcoyle en $C_1$ à $C_6$, on fait réagir un ester de la formule (V)

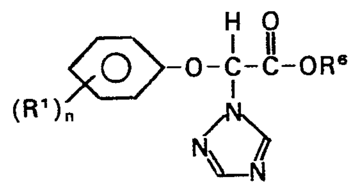 (V)

dans laquelle $R^1$ et n possèdent la signification définie plus haut et $R^6$ représente un radical alcoyle en $C_1$ à $C_4$, d'abord avec un réactif de Grignard de la formule (VI)

$$R^5-Mg-Hal \quad (VI)$$

dans laquelle $R^5$ possède la signification définie plus haut et Hal représente un atome de chlore, de brome ou d'iode, en présence d'un solvant inerte, puis on soumet le carbinol obtenu à une alcoylation selon le mode opératoire a).

4. Fongicide contenant un éther de carbinol suivant la revendication 1 ou suivant la revendication 2.

5. Fongicide contenant un véhicule solide ou liquide et un éther de carbinol suivant la revendication 1 ou la revendication 2.

6. Procédé de préparation d'un fongicide, caractérisé en ce que l'on mélange un véhicule solide ou liquide avec un éther de carbinol suivant la revendication 1.

7. Procédé de lutte contre les champignons, caractérisé en ce que l'on traite les champignons ou les objets à protéger des champignons avec un éther de carbinol suivant la revendication 1.

**Revendications pour l'Etat contractant: AT**

1. Fongicide contenant un éther de carbinol de la formule (I)

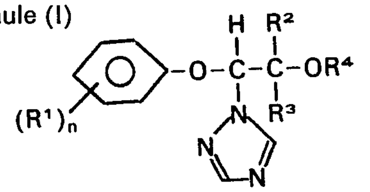 (I)

dans laquelle

$R^1$ désigne un atome d'halogène ou un groupe phényle,

$R^2$ un radical alcoyle en $C_1$ à $C_6$ ou un groupe phényle éventuellement substitué par du chlore,

$R^3$ un radical alcoyle en $C_1$ à $C_6$ ou un groupe vinyle, éthynyle ou benzyle éventuellement substitué par du chlore,

$R^4$ un radical alcoyle en $C_1$ à $C_4$, un groupe allyle, un groupe benzyle éventuellement substitué par du chlore ou le groupe 3,3-diméthylbutane-2-one-1-yle et

n vaut 0, 1 ou 2,

ou un de ses sels ou complexes avec des métaux.

2. Fongicide suivant la revendication 1, contenant un éther de carbinol choisi dans le groupe formé par:

l'éther [1-(1',2',4'-triazol-1'-yl)-1-(4'-chlorophénoxy)-2,3,3-triméthylbutyl-2]-méthylique,

l'éther [1-(1',2',4'-triazol-1'-yl)-1-(4'-chlorophénoxy)-2,3,3-triméthylbutyl-2]-allylique, et

l'éther [1-(1',2',4'-triazol-1'-yl)-1-(4'-chlorophénoxy)-2-vinyl-3,3-diméthylbutyl-2]-méthylique.

3. Procédé de préparation d'un éther de carbinol suivant la revendication 1, caractérisé en ce que:

a) on fait réagir un carbinol de la formule (II)

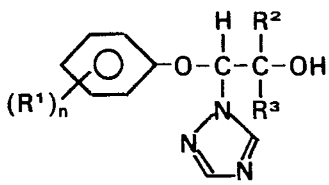 (II)

dans laquelle $R^1$, $R^2$, $R^3$ et n possèdent les significations définies dans la revendication 1, ou un sel de métal alcalin ou d'ammonium d'un tel carbinol, avec un agent d'alcoylation de la formule (III)

$$L-R^4 \qquad (III)$$

dans laquelle $R^4$ possède la signification définie plus haut et L représente un groupe pouvant être éliminé par un clivage nucléophile, à des températures entre 0 et 100°C, le cas échéant dans un solvant ou diluant et éventuellement en présence d'une base inorganique ou organique et/ou en présence d'un accélérateur de la réaction, ou

b) dans le cas de la préparation d'un éther de carbinol suivant la revendication 1 avec $R^2=R^3=R^5=$ un radical alcoyle en $C_1$ à $C_6$,

on fait réagir un ester de la formule (V)

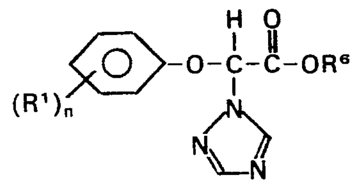 (V)

dans laquelle $R^1$ et n possèdent la signification définie plus haut et $R^6$ représente un radical alcoyle en $C_1$ à $C_4$, d'abord avec un réactif de Grignard de la formule (VI)

$$R^5-Mg-Hal \qquad (VI)$$

dans laquelle $R^5$ possède la signification définie plus haut et Hal représente un atome de chlore, de brome ou d'iode, en présence d'un solvant inerte, puis on soumet le carbinol obtenu à une alcoylation selon le mode opératoire a).

4. Fongicide contenant un véhicule solide ou liquide et un éther de carbinol suivant la revendication 1.

5. Procédé de préparation d'un fongicide, caractérisé en ce que l'on mélange un véhicule solide ou liquide avec un éther de carbinol suivant la revendication 1.

6. Procédé de lutte contre les champignons, caractérisé en ce que l'on traite les champignons ou les objets à protéger des champignons avec un éther de carbinol suivant la revendication 1.